# EUROPEAN PATENT SPECIFICATION

(11) **EP 3 506 877 B1**
(45) Date of publication and mention of the grant of the patent: **27.03.2024**
(21) Application number: 16784566.8
(22) Date of filing: 01.09.2016
(51) Int. Cl.: A61K 8/44, A61Q 5/00, A61K 8/46, A61K 8/88

(54) **CONCENTRATE FOR KERATIN FIBER TREATMENT COMPOSITIONS**
KONZENTRAT FÜR ZUSAMMENSETZUNGEN ZUR BEHANDLUNG VON KERATINFASERN
CONCENTRE POUR COMPOSITIONS DE TRAITEMENT CAPILLAIRE

(43) Date of publication of application: 10.07.2019
(73) Proprietor: Laboratoire Biosthetique Kosmetik GmbH & Co. KG, 75179 Pforzheim (DE)
(72) Inventor: AUGENSTEIN, Gunther, 75179 Pforzheim (DE); ADER, Christian, 75179 Pforzheim (DE)
(74) Representative: Keil & Schaafhausen Patentanwälte PartGmbB
(86) International application number: PCT/IB2016/055223
(87) International publication number: WO 2018/042228

(56) References cited:
- US-A1- 2014 261 518
- DATABASE GNPD [Online] MINTEL; 1. April 2013 (2013-04-01), "Nature's Gate Conditioner", XP002764173, Database accession no. 2050054
- DATABASE GNPD [Online] MINTEL; 1. Februar 2011 (2011-02-01), "Sulfate free Hydrating Shampoo", XP002764174, Database accession no. 1492725
- DATABASE WPI Week 200440 Thomson Scientific, London, GB; AN 2004-423532 XP002764175, & JP 2004 131429 A (KURODA JAPAN KK) 30. April 2004 (2004-04-30)
- DATABASE WPI Week 200444 Thomson Scientific, London, GB; AN 2004-462608 XP002764176, & JP 2004 075630 A (ESTATE CHEM KK) 11. März 2004 (2004-03-11)

## Description

### FIELD OF THE INVENTION

The present invention is a versatile cosmetic composition in form of a liquid concentrate which is useful as additive or ingredient for conventional keratin fiber treatment compositions. It can be used as ready to use additive to conventional yet damaging treatments to which it is added at the time of the treatment. Such treatments are typically hair bleaches, hair colorants or permanent wave compositions. The concentrate comprises a combination of ingredients intended to reduce damage during treatments and/or to prevent undesired deposits on fibers. It is further useful as additive or ingredient for a keratin fiber caring or repairing composition to reduce damage caused by prior treatments and/or reduce undesired deposits on fibers by using the concentrate directly after treatments causing such deposits.

### BACKGROUND OF THE INVENTION

Keratin fibers such as hair consists of protein chains composed of amino acids. These protein chains, which are natural macromolecules, are bound to each other via various types of bonding, such as
a) hydrogen bonding
b) salt bridges between acid and base groups,
c) disulfide bonds.

The quality of keratin fibers, notably human hair, resists many conventional adverse conditions such as shampooing, drying and combing usually quite well. However other exposures to external conditions like sunlight, a variety of environmental factors and harsh treatments, such as exposure to oxidative substances, but also the number and frequency of conventional treatments can negatively affect the quality of hair.

When hair gets wet, water molecules penetrate into the keratin fibers, water reversibly cleaves the hydrogen bonds and hair is swelling. If an acid was added to the water, basic groups in the hair are protonated; if a base was added, acidic groups are deprotonated. In both cases salt bridges are disrupted and due to increased anionic groups at high pH and increased cationic groups at low pH hair swelling is much stronger compared to a pH closer to the neutral region. The strongest bonds which are the disulfide bonds, however, can still hold the protein chains together in the hair fibers under such conditions. This cross-linking by disulphide linkages between the keratin chains accounts for much of the strength of the hair.

Disruption of hydrogen bonds is the first step for all treatments where aqueous compositions are applied, for instance occurs if hair gets wet, e.g. when shampooing. Reconfiguration of the hydrogen bonds is achieved by drying the hair.

Disruption of both, hydrogen bonds and salt bridges, occurs, e.g. by applying hair bleaching or coloring compositions comprising alkalizers such as ammonia, ethanolamine or other alkalizing compounds. After processing, a neutralizing shampoo is applied to rebuild the salt bridges and after drying the previous hair structure can be recovered.

According to US9095518B2 and the reference cited therein (Dombrink et al., Chem Matters, 1983, page 8) hair can already be damaged by weakly alkaline treatments such as slightly alkaline shampoos. The damage is irreversible as with each treatment more and more disulfide bonds are broken, leading to changes in the outer surface of the hair and leaves the hair in a tangled and unmanageable state. The damage caused by alkaline compositions starts with a deprotonation of the cystine group as outlined by structure (I). The resulting structure (II) is unstable and a sulfur atom is lost to give structure (III).

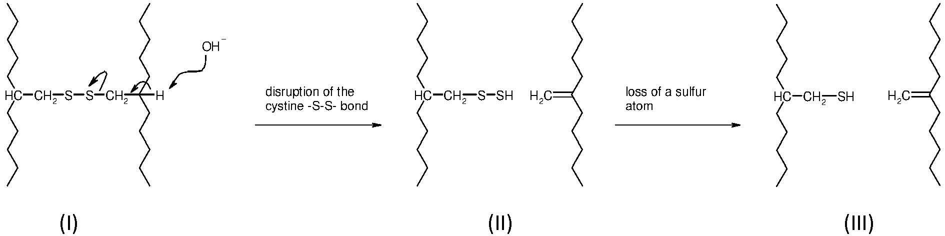

Side reactions of structure (III) may include a recombination with the locally opposed double bond to form a lanthionine group; this mechanism is known from the application of lye straighteners. Or the cysteine moiety of structure (III) can be oxidized to form cysteic acid since most of the strongly alkaline treatments are oxidative treatments such as bleach and dyeing compositions.

In waving or straightening compositions thiols are used with the purpose of braking cystin groups in order to reconfigure the hair to the desired shape.

US9095518B2 claims a repair system in which the liberated cysteine unit of (III) should react with maleic or fumaric acid anions; by ionic interaction with an added basic bridging group such as bis-aminopropyl diglycol hair should be stabilized. Finally, the claimed mechanism is durably modifying previously damaged hair, however without recovering the original hair structure which result cannot be satisfactory in light of the intention to re-establish the previous status quo.

Besides the above described undesired disruption of disulfide bonds by alkaline treatments various permanent waving techniques require the intentional and targeted disruption of a sufficient number of disulfide bonds in order to reshape the hair fibers. After reconfiguration of the hair, either straightening or curling, the disulfide bonds are reconstituted by an oxidative step, e.g. by immersing the hair with a diluted hydrogen peroxide composition. While this intends to reconfigure disulfide bonds from previously generated free cysteine groups the oxidative treatment is far from adequate and often far too late from catching sufficient disrupted disulphide bonds and hence leaves substantial number of damaged sites in the fibers.

The inconvenience of all hair treatments which require conditions at higher pH is that once disruption of disulfide bonds has occurred side reactions may lead to the formation of functional groups which cannot chemically interact with each other so that reconfiguration of the cystine bonds is prevented. Hence, in such cases the hair structure is irreversibly weakened.

Another type of inconveniences of general nature is that during treatments such as shampooing, dyeing, bleaching or permanent waving various active substances soluble in water penetrate into the hair fiber and may form deposits which are hardly soluble and consequently hair feels coarse. Further, the quality of water which is used for shampooing and rinsing the hair may significantly contribute to its roughness. The hardness of water can become an issue as predominantly calcium ions may precipitate numerous anions to form poorly soluble salts. If calcium is in contact with sulfates or persulfates which are commonly used in bleach compositions deposits may persist for a very long time and give the feel of roughness of hair. But in case of carbonates from a water based calcium source an acidic treatment can efficiently dissolve deposits on hair as carbonates are unstable under acidic conditions. Of course this does then require an additional treatment step or change of established treatment conditions.

From JP 2004131429 A a pretreatment hair protecting agent comprising 0.1 to 10 wt.-% of a copolymer that contains a cystine unit or derivative thereof such as Crodasone Cysteine is known. Further 0.01 to 1.0 wt.-% of a reducing agent such as sodium sulfite or L-cysteine may be contained. The to-be-treated hair is first coated with the pretreatment agent at 36°C for 10 min. Subsequently, an oxidative hair dye is applied.

### SUMMARY OF THE INVENTION

The focus of the current invention is therefore a system preventing the formation of undesired deposits on hair and/or limiting/reducing hair damages due to permanently disrupted disulfide bonds and/or even repairing such damaged hair.

Conventional hair treatments which work at high pH and can chemically damage the keratin macromolecules are hair colorants, bleaches and permanent waving treatments or as indicated above mildly alkaline conventional treatments. It was found that a composition comprising at least
- a thiol, and
- a polymer or copolymer comprising cystine units, said polymer or copolymer being Cystine Bis-PG-Propyl Silanetriol
efficiently protects the hair against damages normally caused by such alkaline treatments.

Prior to treating hair the concentrate composition of the current invention is added to a ready-to-use mixture of hair colorants, hair bleaches or permanent wave lotion and thereby applied to protect the hair during an alkaline treatment. Alternatively an inventive concentrate can also be used in neat or diluted form or preferably in combination with another treatment regime, after a damaging alkaline treatment. The amount of the concentrate to be used and the amount of the components in the concentrate will vary naturally with the particular intended use. The respective effective amounts have to be chosen such that the desired purpose under the intended usage conditions (additive to a treatment composition or use in diluted or neat form) can be achieved on one hand. On the other hand, while changes to the underlying alkaline treatment might be accepted, it is usual that no such changes are intended, such that the recommended effective amount is conventionally desired to be neutral, i.e. causes no change in result, to the alkaline treatment itself. As an example the concentrate and/or its components should not be employed in an amount changing the pH, color, waving/straitening or bleaching result in a noticeable way.

### BRIEF DESCRIPTION OF THE FIGURES:

Figure 1 shows a 1000 times electron microscope enlargement of a hair fiber after the hair fiber was 3 times bleached in a conventional way, making deposits on the hair fiber visually discernible.
Figure 2 shows a 5000 times electron microscope enlargement of the hair fiber of Figure 1, magnifying the visually discernible deposits.
Figure 3 shows a 500 times electron microscope enlargement of a hair fiber after the hair fiber was 3 times bleached in a conventional way using the inventive concentrate as additive in the bleaching composition, not showing any visually discernible deposits.
Figure 4 shows a 3200 times electron microscope enlargement of the hair fiber of Figure 3 confirming the absence of visually discernible deposits.

### DETAILED DESCRIPTION OF THE INVENTION

The thiol of the present invention is a reducing agent with at least one -SH group. Thiols are well known from waving and straightening compositions to break disulfide bonds of the cystine groups in the hair. In contrast thereto thiols as ingredients of the present invention have surprisingly been found to contribute the protection the hair structure.

The thiol reducing agent can preferably be chosen from the group consisting of thioglycolic acid and derivatives thereof, in particular esters thereof such as glycerol- or glycolmonothioglycolate; thiolactic acid and derivatives thereof, in particular esters thereof such as glycerol monothiolactate; 3-mercaptopropionic acid and derivatives thereof, in particular esters thereof such as glycerol 3- mercaptopropionate and ethyleneglycol 3-mercaptopropionate; cysteamine and derivatives thereof, in particular acyl derivatives thereof such as N-acetylcysteamine and N-propionylcysteamine; mono- thioglycerol and derivatives thereof, in particular esters thereof; cysteine and derivatives thereof, in particular the amino acid cystein and its salts or esters such as N-acetylcysteine esters, N- alkanoylcysteine esters and cysteine alkyl esters and their salts.

The thiol reducing agent should be used in the desired effective amount it is typically used in a total lower concentration by weight of the concentrate from about 0.01 %, preferably from 0.1%, more preferably from 1.0% and at an upper concentration by weight of the concentrate of up to 10%, preferably up to 5.0% and more preferably up to 4.0%.

Suitable polymers or co-polymers of the current invention should be used in the desired effective amount as described above. Such polymers or co-polymers should include local structures of cystine for the repair or damage prevention or for the deposit reduction according to the present invention.. , According to the invention, the cystine source is a compound having the INCI name Cystine Bis-PG-Propyl Silanetriol, available e.g. from Croda GmbH, Herrenpfad-Sud 33, 41334 Nettetal Kaldenkirchen, Germany.

Typically the polymer and/or copolymer comprising at least one cystine unit is present at a lower concentration by weight of the concentrate should be from about 0.1%, preferably from 0.5% and at an upper concentration by weight of the concentrate of up to 3%, preferably up to 1.5%. Useful levels of the Cystine Bis-PG-Propyl Silanetriol have been found to be in the range from 0.5% to 1.5% by weight, relative to the total weight of the concentrate.

### Other ingredients and characteristics

The inventive composition may further comprise a reducing agent with no thiol group. The non-thiol reducing agent may preferably be chosen from the group consisting of sulfites, bisulfites, sulfamates, phosphines, sugars, reductones and combinations thereof. More preferably, the non-thiol reducing agent may be selected from ammonium sulfites and bisulfites as well as alkali metal sulfites and bisulfites, and more preferably sodium sulfite and sodium bisulfite.

The non-thiol reducing agents are used in at a total concentration of from 0.01 to 10% by weight, preferably from 0.01 to 5.0% by weight, more preferably from 0.1 to 2.0% by weight, and even more preferably from 0.1 to 1.0% by weight, relative to the total weight of the concentrate composition.

While the concentrate according to the present invention can have a pH in the range from 2 to 12 across the whole spectrum of application situations, it has been found for typical alkaline application situations that a pH from 6 to 10, preferably from 7.5 to 9.5 provides a good starting point to ensure a performance neutral concentrate. In order to adjust the pH one or more acidic or alkali agents may be used alone or in combination. As the acidic agents, mention may be made of any inorganic or organic acids which are commonly used in cosmetic products such as citric acid, lactic acid, phosphoric acid or sulfuric acid. As the alkali agents, mention may be made of any inorganic or organic basic agents which are commonly used in cosmetic products such as ammonia; alkanolamines such as mono and triethanolamine, isopropanolamine; 2-Amino-2-methyl-1-propano (AMP), 2-amino-2-hydroxymethyl-propane-1,3-diol (Tris), sodium and potassium hydroxides; guanidine and combinations or derivatives thereof.

The amount of the acidic or alkali agent(s) is not limited, but may be from 0.1 to 5% by weight relative to the total weight of the composition.

The cosmetic concentrate composition used in the present invention preferably comprises water and may advantageously contain one or several organic solvents, which particularly include alcohols, such as ethyl alcohol, isopropyl alcohol, benzyl alcohol and phenylethyl alcohol, or polyols such as propylene glycol, butylene glycol, hexylene glycol and glycerin; or polyol ethers, such as ethylene glycol monomethyl, monoethyl and monobutyl ethers, propylene glycol ethers such as propylene glycol monomethylether, butylene glycol, dipropylene glycol as well as diethylene glycol alkyl ethers, such as diethylene glycol monoethylether or monobutylether.

Water would usually be present to complete the composition to a full 100% by weight relative to the total weight of the concentrate composition which often should be in a concentration of from 50% to 90% by weight relative to the total weight of the concentrate composition. The organic solvent(s) may then be present in an amount of from 0.1% to 10%, and preferably from 1% to 5% by weight relative to the total weight of the composition.

The compositions may contain synthetic or natural waxes, and especially fatty alcohols preferentially of from 12 to 18 carbon atoms such as lauryl, myristyl, cetyl and stearyl alcohol and mixtures thereof. The amount may vary in a range up to 10%, preferably up to 6% by weight relative to the total weight of the concentrate composition

The concentrate composition may contain one or several surfactants such as anionic, amphoteric, zwitterionic, nonionic, cationic, or combinations thereof.

The anionic surfactant can be any of the anionic surfactants known or previously used in the art of aqueous surfactant compositions. Suitable anionic surfactants include but are not limited to alkyl sulfates, alkyl ether sulfates, alkaryl sulfonates, alkyl succinates, alkyl sulfosuccinates, N-alkoyl sarcosinates, alkyl phosphates, alkyl ether phosphates, alkyl ether carboxylates, alkylamino acids, alkyl peptides, alkoyl taurates, carboxylic acids, acyl and alkyl glutamates, alkyl isethionates, and alpha-olefin sulfonates, especially their sodium, potassium, magnesium, ammonium and mono-, di- and triethanolamine salts. The alkyl groups generally contain from 8 to 18 carbon atoms and may be unsaturated. The alkyl ether sulfates, alkyl ether phosphates and alkyl ether carboxylates may contain from 1 to 10 ethylene oxide or propylene oxide units per molecule, and preferably contain 1 to 3 ethylene oxide units per molecule.

Examples of suitable anionic surfactants include sodium, ammonium, and triethanolamine lauryl sulfate, sodium and ammonium lauryl ether sulfate (with 1, 2, and 3 moles of ethylene oxide), disodium laureth sulfosuccinate, sodium cocoyl isethionate, sodium C12-14 olefin sulfonate, sodium laureth-6 carboxylate, sodium C12-15 pareth sulfate, sodium methyl cocoyl taurate, sodium dodecylbenzene sulfonate, sodium cocoyl sarcosinate, triethanolamine monolauryl phosphate, and fatty acid soaps.

The nonionic surfactant can be any of the nonionic surfactants known or previously used in the art of aqueous surfactant compositions. Suitable nonionic surfactants include but are not limited to aliphatic (C6-C18) primary or secondary linear or branched chain acids, alcohols or phenols, alkyl ethoxylates, alkyl phenol alkoxylates (especially ethoxylates and mixed ethoxy/propoxy), block alkylene oxide condensate of alkyl phenols, alkylene oxide condensates of alkanols, ethylene oxide/propylene oxide block copolymers, semi-polar nonionics (e.g., amine oxides and phospine oxides), as well as alkyl amine oxides. Other suitable nonionics include mono or di alkyl alkanolamides and alkyl polysaccharides, sorbitan fatty acid esters, polyoxyethylene sorbitan fatty acid esters, polyoxyethylene sorbitol esters, polyoxyethylene acids, and polyoxyethylene alcohols.

Examples of suitable nonionic surfactants include coco monoethanolamide, coco diglucoside, alkyl polyglucoside, cocamidopropyl and lauramine oxide, polysorbate 20, the alkoxylated linear alcohols cetearteh-12 and ceteareth-20, laureth-9, trideceth-9, glyceryl stearate, PEG-100 stearate, and oleth 20.

Amphoteric and zwitterionic surfactants are those compounds which have the capacity of behaving either as an acid or a base. These surfactants can be any of the surfactants known or previously used in the art of aqueous surfactant compositions. Suitable materials include but are not limited to alkyl betaines, alkyl amidopropyl betaines, alkyl sulphobetaines, alkyl glycinates, alkyl carboxyglycinates, alkyl amphopropionates, alkyl amidopropyl hydroxysultaines, acyl taurates and acyl glutamates wherein the alkyl and acyl groups have from 8 to 18 carbon atoms.

Examples include cocamidopropyl betaine, sodium cocoamphoacetate, cocamidopropyl hydroxysultaine, and sodium cocamphopropionate.

The cationic surfactants can be any of the cationic surfactants known or previously used in the art of aqueous surfactant compositions. Suitable cationic surfactants include but are not limited to alkyl amines, alkyl imidazolines, ethoxylated amines, quaternary compounds thereof, and quaternized esters. In addition, alkyl amine oxides can behave as a cationic surfactant at a low pH.

Examples include lauramine oxide, dicetyldimonium chloride, behentrimonium methosulfate, cetrimonium methosulfate and cetrimonium chloride.

The cosmetic concentrate composition of the present invention may also comprise at least one additional ingredient. The amount of the additional ingredient(s) is not limited, but may be from 0.01 to 20% by weight relative to the total weight of the concentrate composition. The additional ingredient(s) may be selected from the group consisting of volatile or non volatile, linear or cyclic, amine-type or not, silicones, cationic, anionic, non ionic or amphoteric polymers, peptides and derivatives thereof, viscosity regulating agents and penetrating agents, as well as other active compounds, agents for combating hair loss, anti-dandruff agents, deodorizing agents, associative- type or not, natural or synthetic thickeners, suspending agents, sequestering agents, opacifying agents, dyes, sunscreen agents, fillers, vitamins or provitamins; mineral, vegetable or synthetic oils, as well as fragrances, preserving agents, stabilizers, and mixtures thereof.

The inventive composition may further comprise keratin building blocks such as oligopeptides selected from a di, tri or tetrapeptide, hydrolyzed keratin, a hydrolyzate of a phytoprotein isolated from soy, wheat or oat, a silk protein or hydrolyzed and mixtures thereof.

The amount of compounds having a peptide structure may be of from 0.1% to 5% by weight, preferentially from 0.5% to 1.5% by weight relative to the total weight of the concentrate composition.

In addition to a polymer or copolymer comprising cystine units being Cystine Bis-PG-Propyl Silanetriol the cosmetic concentrate composition may comprise further hair conditioning silicones such as silanetriols, wherein the polymer is selected from the group consisting of hydrolyzed vegetable protein PG-Propyl Silanetriol, hydrolyzed wheat protein PG-Propyl Silanetriol.

Additional silicone compounds suitable for being used in the formulations include, but are not limited to a silicone compound, for instance dimethicones, dimethicone polyols, and dimethicone esters. For example, in one embodiment, the cosmetic concentrate composition includes PEG-12 dimethicone, sold under the trade name Dow Corning 193 Fluid Dow Corning GmbH at Rheingaustraße 34, D-65201 Wiesbaden, Germany.

Aspects of typical use of the invention:
The cosmetic concentrate composition used in the present invention may be in any form desired such as a liquid, a lotion, a spray, a gel, a foam, or a cream - this will depend strongly on convenience for the specifically desired application. For alkaline type treatments the hair is treated conventionally without changing the general processing of applying the color, the bleach and the permanent wave composition, except that either the inventive composition is added to this treatment composition or used in diluted form afterwards. In order to prevent or limit hair from being damaged in the course of an alkaline treatment the concentrate composition is added to a ready-to-use mixture of a hair color, a bleach composition, or a permanent wave lotion, in an amount of from 2% to 10% by weight to the coloring or bleaching composition and of from 4% to 35% by weight of the permanent wave lotion as according to the invention.

Damages to hair are visible for instance after bleaching or coloring the hair where hair is porous, feels coarse and has no shine. Many specialists in the art of hair treatments try to rebuild the sensational hair quality by the application of various products such as oil treatments. However, the current invention is very useful especially after bleaching or coloring to regain some or all of the natural reasons for natural shine and softness of the hair. In such case where hair is already damaged the inventive concentrate composition can be diluted, preferably with water or added to a post alkaline treatment composition, in a ratio of from 1:2 up to 1:8, preferred is a ratio of from 1:5 to 1:7 and then applied to the damaged hair.

The pH of the composition after mixing with the colorant, the bleach or the perm is generally between 5.5 and 10.5 and preferably between 6 and 10.

The following examples illustrate the current invention but the invention is not limited to these examples.

### EXAMPLES

### Example 1: Concentrate Composition for adding to bleaches, colorants and permanent wave lotions according to the invention

In all examples the phrase 'add up to 100%' is used to indicate that the concentration of water is such that it adds up to 100% of the respective formulation, whether concentrated or diluted. The other ingredients are added in the concentration indicated based on their commercial source.

### Example 1a) Clear lotion

| | |
|---|---|
| Cysteine HCl | 3.00 % |
| Cystine Bis-PG Propyl Silanetriol | 0.80 % |
| Hydrolyzed Keratin | 0.90% |
| Trideceth-10 | 1.10% |
| PEG-12 Dimethicone | 5.00 % |
| PEG-180/Laureth-50/TMMG Copolymer | 0.40% |
| Alcohol denat. | 0.80% |
| Hexylene Glycol | 0.45 % |
| PEG-40 Hydrogenated Castor Oil | 1.00 % |
| Sodium Sulfite | 0.25% |
| Sodium Hydroxide | 1.55% |
| Tetrasodium EDTA | 0.10% |
| Fragrance | 0.45 % |
| Water | add up to 100.00 % |

### Example 1b) Opaque lotion

| | |
|---|---|
| Cysteine HCl | 3.00% |
| Cystine Bis-PG Propyl Silanetriol | 1.20% |
| Alcohol denat | 1.20% |
| Hydrolyzed Keratin | 1.10% |
| Dimethicone | 2.20% |
| Xanthan Gum | 0.25% |
| PEG-40 Hydrogenated Castor Oil | 1.00% |
| Sodium Sulfite | 0.25% |
| Sodium Hydroxide | 1.55% |
| Tetrasodium EDTA | 0.10% |
| Fragrance | 0.50% |
| Phenoxyethanol | 0.90% |
| Cetearyl Alcohol | 5.50% |
| Dicaprylyl Ether | 2.80% |
| Ceteareth-30 | 2.70% |
| Ceteareth-12 | 1.00% |
| Butylene Glycol | 0.15% |
| Water | add up to 100.00 % |

### Example 1c) Clear lotion

| | |
|---|---|
| Thiolactic Acid | 4.50% |
| Cystine Bis-PG Propyl Silanetriol | 0.80% |
| Alcohol denat | 0.80% |
| Hydrolyzed Wheat Protein | 0.90% |
| PEG-12 Dimethicone | 3.00% |
| Xanthan Gum | 0.40% |
| PEG-40 Hydrogenated Castor Oil | 1.00% |
| Sodium Sulfite | 0.25% |
| Sodium Hydroxide | 1.55% |
| Tetrasodium EDTA | 0.10% |
| Fragrance | 0.45% |
| Phenoxyethanol | 0.50% |
| Water | add up to 100.00 % |

The compositions of Example 1a)-c) have each a pH of between 9.0 - 9.5.

### Example 1d) Clear diluted lotion (Rebuild treatment)

Example 1d) may be prepared by dilution of Example 1 a), e.g. by dilution of 4 ml of the composition described by Example 1a) and 25 ml water at a ratio of 1:6.25.

| | |
|---|---|
| Cysteine HCl | 0.480 % |
| Cystine Bis-PG Propyl Silanetriol | 0.128 % |
| Hydrolyzed Keratin | 0.144 % |
| Trideceth-10 | 0.176 % |
| PEG-12 Dimethicone | 0.800 % |
| PEG-180/Laureth-50/TMMG Copolymer | 0.064 % |
| Alcohol denat. | 0.128 % |
| Hexylene Glycol | 0.450 % |
| PEG-40 Hydrogenated Castor Oil | 0.076 % |
| Sodium Sulfite | 0.040 % |
| Sodium Hydroxide | 0.248 % |
| Tetrasodium EDTA | 0.016 % |
| Fragrance | 0.071 % |
| Water | add up to 100.000 % |

The pH of 1d) is between 8.7- 9.2.

This composition is added to dry hair. After a processing time of 2 to 10 minutes, the hair is rinsed, washed with a shampoo and can optionally be further treated with the compositions described in Example 2.

### Example 2: Neutralizing and conditioning compositions

(Example 2 is not an embodiment of the invention but can be used advantageously together with the invention).

### Example 2a): Spray

The following composition is used to neutralize the hair after a bleach, a color or a perm waving treatment.

| | |
|---|---|
| Cysteine HCl | 0.01% |
| Hydrolyzed Keratin | 0.02% |
| Hydrolyzed Wheat Protein | 1.10% |
| Panthenol | 1.00% |
| Magnesium Sulfate | 4.62% |
| Cetrimonium Chloride | 1.25% |
| Trideceth-9 | 0.32% |
| Disodium Cocoamphoacetate | 0.40% |
| PEG-40 Hydrogenated Castor Oil | 0.32% |
| Propylene Glycol | 0.08% |
| Phenoxyethanol | 0.89% |
| Sodium Benzoate | 0.05% |
| Fragrance | 0.30% |
| Water | add up to 100.00% |

The composition is a clear liquid and the pH is between 4.8 - 5.2. The liquid is preferentially used as a pump spray to neutralize the treated hair and as nourishing composition. The hair is left for approximately 10 minutes followed by treating with a hair mask of Example 2b).

### Example 2b): Hair mask

The hair mask is added after the spray.

| | |
|---|---|
| Cetearyl Alcohol | 5.60% |
| Propylene Glycol Dibenzoate | 5.00% |
| Propylene Glycol | 3.75% |
| Cyclopentasiloxane | 3.70% |
| Quaternium-91 | 1.25% |
| Cetrimonium Methosulfate | 1.75% |
| Dimethiconol | 0.30% |
| Hydrolyzed Wheat Protein | 0.50% |
| Moringa Pterygosperma Seed Extract | 0.50% |
| Amidimethicone | 0.95% |
| Hydroxypropyl Guar Hydroxypropyltrimonium Chloride | 0.25% |
| Phenoxyethanol | 0.02% |
| Sodium Benzoate | 0.02% |
| Fragrance | 0.30% |
| Ceramide 2 | 0.30% |
| Tromethamine | 0.09% |
| Water | add up to 100.00% |

The composition is of a creamy consistency and the pH is between 3.2 - 3.4.

The hair mask composition is applied after the spray of Example 2a) and provides a long lasting moisturized feel and smooth feel to the hair, without feeling greasy.

### Example 2c): Nourishing hair mask

| | |
|---|---|
| Hydroxypropyl Guar Hydroxypropyltrimonium Chloride | 0.27% |
| Cysteine HCl | 0.05% |
| Hydrolyzed Keratin | 2.50% |
| Panthenol | 0.80% |
| Magnesium Sulfate | 4.25% |
| Cetrimonium Methosulfate | 1.75% |
| Propylene Glycol Dibenzoate | 5.00% |
| Quaternium-91 | 5.25% |
| Cetearyl Alcohol | 5.73% |
| Ceteareth 30 | 3.00% |
| Ceteareth 12 | 1.00% |
| Cyclopentasiloxane | 3.70% |
| Dimethiconol | 0.30% |
| Moringa Pterygosperma Seed Extract | 0.50% |
| Amidimethicone | 0.93% |
| Phenoxyethanol | 0.02% |
| Sodium Benzoate | 0.02% |
| Fragrance | 0.30% |
| Citric Acid | 0.02% |
| Water | add up to 100.00% |

The composition of Example 2c) combines both in one, the neutralizing composition 2a) and the hair mask 2b) and gives the moisturized and smooth feel to the hair as described herein.

### Example 3: Color altering treatments

Prior to the experiments using hair color compositions with and without the use of the present invention the amino acids of hair fibers was analyzed first for virgin hair (no damage) and then for hair treated with the inventive composition (nothing to repair, hence expected not have a substantive effect). The results are given by the following table. Prior to the test, the hair swatches were washed with a clarifying shampoo.

### 3a): Virgin human hair

### 3b): Virgin human hair was treated with the inventive composition

| Example | 3a) | 3b) |
|---|---|---|
| Amino acid | Virgin hair | Treated with the inventive composition |
| Cystine | 596.0 | 594.6 |
| Isoleucine | 198.7 | 198.1 |
| Leucine | 465.7 | 466.9 |
| Tyrosine | 143.0 | 146.9 |
| Phenylalanine | 122.2 | 123.1 |
| Ornithine | 8.0 | 9.1 |
| Lysine | 178.6 | 177.3 |
| Histidine | 56.8 | 55.3 |
| Arginine | 480.6 | 474.2 |
| Asparagine | 403.6 | 404.7 |
| Threonine | 534.2 | 530.9 |
| Serine | 825.2 | 823.4 |
| Glutamine | 937.7 | 944.5 |
| Proline | 581.5 | 575.4 |
| Glycine | 434.9 | 437.3 |
| Alanine | 344.0 | 348.3 |
| Valine | 388.2 | 391.1 |
| Methionine | 35.4 | 35.5 |
| Total [µmol/g] | 6734.3 | 6736.6 |

The analysis for all amino acids present in keratin fibers of virgin human hair is listed in column 3a). The sum of all amino acids is 6734.3 µmol/g hair.

The application of the inventive composition to virgin hair is given by column 3b). The amount of the individual amino acids has practically not changed and the sum of 6736.6 µmol/g corresponds to the reference, indicating that the inventive composition by itself does not affect the quality of hair.

### Bleach Composition

A non-inventive composition of a bleach comprising the following components was used:

| | |
|---|---|
| Potassium Persulfate | 36.40% |
| Ammonium Persulfate | 19.40% |
| Sodium Metasilicate | 11.80% |
| Magnesium Carbonate Hydroxide | 9.60% |
| Sodium Stearate | 8.60% |
| Guar Gum | 4.90% |
| Paraffinum Liquidum | 7.00% |
| Sodium Lauryl Sulfate | 0.90% |
| Hydrolyzed Wheat Protein | 0.90% |

| | |
|---|---|
| Tetrasodium EDTA | 0.50% |
| Total | 100.00% |

The bleach composition corresponds to a typical bleach powder composition which is commercially available and mixed at a ratio of 1:1 to 1:2 with a commercial developer comprising essentially 40% by weight concentration of peroxide and other conventional ingredients (fragrance etc.).

In order to compare the effect of the inventive composition in a bleach tests have been performed wherein the above described composition was processed by a standard procedure vs. a composition comprising same bleach composition to which the inventive composition was added.

### Hair swatches 3x conventionally bleached

| Treatment | |
|---|---|
| 1 x | washing with a clarifying shampoo |
| | drying the hair swatch |
| 3 x | mixing 10 g bleach powder with 20 g developer 40 Vol. |
| | applying to the hair |
| | processing for 40 minutes at ambient temperature (no heat) |
| | rinsing the hair |
| | washing with a shampoo |
| | air-drying of the hair sample |

### Hair swatches 3x bleached in presence of the inventive composition

| Treatment | |
|---|---|
| 1 x | washing with a clarifying shampoo |
| | drying the hair sample |
| 3 x | mixing 10 g bleach powder with 20 g developer 40 Vol. |
| | adding 1.5 g of the inventive composition from Example 1a) |
| | mixing thoroughly |
| | applying to the hair |
| | processing for 40 minutes at ambient temperature (no heat) |
| | rinsing the hair |
| | washing with a shampoo |

In the following table results are given of the hair analysis after the application of a conventional bleach vs. a conventional bleach comprising the inventive composition.

### 3a): Virgin human hair

### 3c): Virgin human hair bleached in the conventional way

### 3d): Virgin human hair bleached in presence of the inventive composition

| | 3a) | 3c) | 3d) |
|---|---|---|---|
| Amino acid | Virgin hair | Conventional bleach | Conventional bleach + inventive composition |
| Cystine | 596.0 | 400.9 | 433.3 |
| Isoleucine | 198.7 | 183.0 | 197.7 |
| Leucine | 465.7 | 430.3 | 460.1 |
| Tyrosine | 143.0 | 115.0 | 124.4 |
| Phenylalanine | 122.2 | 111.5 | 119.2 |
| Ornithine | 8.0 | 4.9 | 7.8 |
| Lysine | 178.6 | 161.6 | 174.8 |
| Histidine | 56.8 | 47.9 | 51.6 |
| Arginine | 480.6 | 449.0 | 475.6 |
| Asparagine | 403.6 | 385.7 | 411.5 |
| Threonine | 534.2 | 505.1 | 536.2 |
| Serine | 825.2 | 771.2 | 825.2 |
| Glutamine | 937.7 | 889.4 | 956.3 |
| Proline | 581.5 | 538.3 | 575.2 |
| Glycine | 434.9 | 401.0 | 426.1 |
| Alanine | 344.0 | 320.4 | 341.5 |
| Valine | 388.2 | 365.3 | 389.4 |
| Methionine | 35.4 | 22.8 | 24.7 |
| Total | 6734.3 | 6103.3 | 6530.6 |
| [µmol/g] | | | |

A remarkable loss across all amino acids occurs if hair is bleached by a conventional composition; results are listed in column 3c). The result after the bleach carried out in presence of the inventive composition is given by column 3d).

The individual and total amount of Amino acids of column 3d) with a total of 6530.6 µmol/g is substantially higher than the amount obtained without the presence of the inventive composition which is in total only 6103.3 µmol/g.

Notably, the loss calculated in percent is not constant across all amino acids. Values of the amino acid Ornithine for instance drops dramatically, namely from 8.0 µmol/g for the virgin hair to 4.9 µmol/g after a conventional bleach; in presence of the inventive composition, 7.8 µmol/g was analyzed which is close to the level of the virgin hair.

This result is remarkable as the composition of the current invention does not comprise Ornithin. Other amino acids do not drop at such a rate indicating damage of the hair. If the hair was just "filled" with ingredients of the inventive composition, the amino acid analysis would show an equal percentage in the loss across all amino acids.

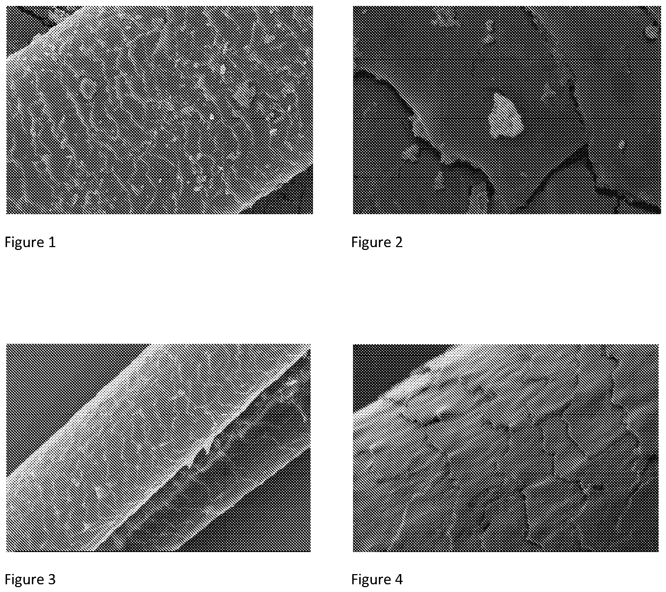

As another aspect the hair was visually analyzed under electron microscope magnification. The resulting pictures can be seen in Figures 1 and 2 for bleached hair and Figures 3 and 4 for hair bleached in the presence of the inventive composition. The pictures are very clear examples how deposits can occur in absence and do not occur in presence of the inventive composition.

The formation of deposits as illustrated by Figures 1 and 2 have most notably been observed if compositions with higher levels of persulfates were used. It is therefore speculated that the deposit is a salt comprising the persulfate or sulfate ion such as calcium sulfate, sourced from the calcium in standard tap water. There are no deposits for the same beach composition to which the inventive concentrate composition was added.

Typically compositions designed for bleaching the hair comprise persulfates in amounts of from 40% to 60% by weight and for this reason deposits have predominantly been observed after bleaching the hair. In hair colorants the amount of sulfates which may interact with calcium ions is considerable lower as most of the sulfates come from dye components such as Toluene-2,5-Diamine Sulfate, 2,4-Diaminophenoxyethanol Sulfate or N,N-Bis(2-hydroxyethyl)-p-phenylenediamine Sulfate. Further, in order to adjust viscosities, alkali or ammonium sulfates may also be present. However, conditions of forming undesired residues in the hair on the surface of hair are also given.

In case of permanent waving compositions the expectation of forming deposits on the hair surface is not as high as for bleaches or colorants. However, permanent waving treatments are known to weaken hair as many of the reduced cystin bonds cannot be rebuilt at the end of the process. The inventive composition is able to improve the hair quality which was confirmed by trained hair stylists experienced in various permanent wave techniques. In this context it should be emphasized that in a professional rating 9 of 10 stylists gave a better rating to hair quality if the inventive composition was added to the permanent wave lotion while the remaining stylist rated equal to the treatment without addition of the inventive composition. The improvements were on the overall quality of the hair, predominantly on the bounce, the suppleness and the shine. A direct comparison to the commercial available product Olaplex* was not possible due to the incompatibility of Olaplex with thiol-group-containing compounds (e.g. thiolactic acid).

The hair treatment which is normally an alkaline treatment is complemented in an outstanding manner by the application of a neutralizing and mineralizing composition which is sprayed to the hair. Furthermore the application of a hair mask optimized for being used as after treatment leads to a highly desirable recovering of hair quality in practical salon treatment regimes.

### Suppleness of bleached hair - conventional vs. inventive

The efficiency of the inventive composition has been further explored by determination of the suppleness of hair. The method to determine suppleness of hair is a proprietary method of the DWI Leibniz-Institut, Aachen/Germany. In this standardized method higher values refer to reduced suppleness. Hair samples conventionally bleached vs. samples bleached in the same way after adding the inventive concentrate composition to the bleach were compared.

| **DWI suppleness values** | **Virgin hair** | **Hair 3x bleached by the conventional bleach composition** | **Suppleness Result** |
|---|---|---|---|
| **Mean ± Stdev [Nm]** | 0.0127 ± 0.0017 | 0.0135 ± 0.0013 | decrease by 6.3% |

The result shows that bleaching hair by using a conventional bleach leads to a hair quality which is harder compared to the hair quality before the treatment.

| **DWI suppleness values** | **Virgin hair** | **Hair 3x bleached by the conventional bleach composition together with the inventive composition** | **Suppleness Result** |
|---|---|---|---|
| **Mean ± Stdev [Nm]** | 0.0126 ± 0.0017 | 0.0109 ± 0.0019 | increase by 13.5% |

Bleaching the hair in a similar way in presence of the inventive composition leads to a considerable increase of suppleness vs. the untreated reference hair sample.

### Testing vs a commercial reference

On 16 model heads bleach tests have been carried out by hair dressers in half-head blind tests comparing on the same head bleach compositions comparing the inventive concentrate vs Olaplex* as a commercial market reference. The hair dressers were not told which composition was reference and which the inventive composition.

Olaplex* was added to the bleach according to the instructions for use to give Composition A. At the same time the above bleach composition comprising the inventive composition was prepared; hair treatments were performed including the after treatment with compositions from Example 2 and followed the steps indicated below:
- washing with a clarifying shampoo
- drying the hair sample
- mixing 10 g bleach powder with 20 g developer 40 Vol and adding 1.5 g (5%) of the inventive composition from Example 1a) for treating one side of each head
- preparing the same bleach composition and adding Olaplex* according to the usage instructions as reference for the other side of the head
- mixing thoroughly for both compositions
- applying to the hair on one side of each head respectively
- processing for 40 minutes at ambient temperature (no heat)
- rinsing the hair
- washing with a shampoo
- towel drying the hair
- spraying composition of Example 2a) onto the hair
- processing for 5 minutes
- adding composition of Example 2b) onto the hair
- processing for 15 minutes
- rinsing the hair
- washing with a shampoo
- air-drying of the hair sample

Results: 63% of the obtained hair quality comprising the inventive composition was rated superior to the hair quality treated in presence of Olaplex*. 13% of the hair quality was rated equal and 25% of the hair quality comprising Olaplex* was rated better than the hair quality treated with the bleach comprising the inventive composition.

* Olaplex is the trademark of a commercially available hair improvement composition. It is widely available and can for instance be obtained from New Flag GmbH, Max-Joseph-Str. 7, 80333 München, Germany.

### Example 4: Hair Color

A conventional composition of a permanent hair color cream of an extra-light blonde shade was used. The composition has the following ingredients:

| | |
|---|---|
| Propylene Glycol | 5.0000% |
| Toluene-2,5-siamine Sulfate | 0.0050% |
| m-Aminophenol | 0.0110% |
| 2-Methyl-5-Aminophenol | 0.0120% |
| Resorcinol | 0.0100% |
| 2,4-Diaminophenoxyethanol Sulfate | 0.0025% |
| N,N-Bis-Hydroxyethyl-p-Phenylenediamine Sulfate | 0.0660% |
| Stearyl Alcohol | 11.6000% |
| Cetyl Alcohol | 4.4000% |
| Lauryl Alcohol | 2.0000% |
| Myristyl Alcohol | 0.8000% |
| Ceteareth-50 | 4.4000% |
| Ethoxydiglycol | 3.0000% |
| PEG-40 Hydrogenated Castor Oil | 0.2000% |
| Ascorbic Acid | 0.2000% |
| Sodium Sulfite | 0.4000% |
| Disodium EDTA | 0.2000% |
| Polyquaternium-22 | 0.2000% |
| Ammonia 25% in water | 15.0000% |
| Ethanolamine | 0.0500% |
| Fragrance | 0.6000% |
| Water | add up to 100.0000% |

| Treatment with conventional colorant | |
|---|---|
| 1 x | washing with a clarifying shampoo |
| | towel-drying the hair swatch |
| 3 x | mixing 10 g hair color composition with 20 g developer 40 Vol. |
| | applying to the hair |
| | processing for 30 minutes at ambient temperature (no heat) |
| | rinsing the hair |
| | washing with a neutralizing shampoo |
| | blow-drying of the hair sample |

| Treatment with conventional colorant combined with the inventive concentrate composition | |
|---|---|
| 1 x | washing with a clarifying shampoo |
| | drying the hair sample |
| 3 x | mixing 10 g hair color composition with 20 g developer 40 Vol. |
| | adding 1.5 g (5%) of the inventive composition from Example 1a) |
| | mixing thoroughly |
| | applying to the hair |
| | processing for 30 minutes at ambient temperature (no heat) |
| | rinsing the hair |
| | washing with a neutralizing shampoo |
| | Blow- drying of the hair sample |

The light blonde hair color thus obtained was identical in both cases.

Amino acid analysis of the colored hair fibers:
4e): The hair was colored in the conventional way 4d): The hair was colored in presence of the inventive composition

| | 4a) | 4e) | 4f) |
|---|---|---|---|
| Amino acid | Virgin hair | Conventional colorant | Conventional colorant + inventive composition |
| Cystine | 596.0 | 508.6 | 526.7 |
| Lsoleucine | 198.7 | 179.9 | 187.8 |
| Leucine | 465.7 | 423.8 | 441.5 |
| Tyrosine | 143.0 | 132.6 | 135.1 |
| Phenylalanine | 122.2 | 109.9 | 113.9 |
| Ornithine | 8.0 | 6.5 | 8.2 |
| Lysine | 178.6 | 163.7 | 168.5 |
| Histidine | 56.8 | 52.0 | 53.8 |
| Arginine | 480.6 | 444.1 | 452.2 |
| Asparagine | 403.6 | 377.4 | 385.2 |
| Threonine | 534.2 | 492.5 | 501.1 |
| Serine | 825.2 | 764.0 | 772.0 |
| Glutamine | 937.7 | 876.3 | 890.0 |
| Proline | 581.5 | 524.8 | 544.4 |
| Glycine | 434.9 | 401.0 | 409.2 |
| Alanine | 344.0 | 317.6 | 325.2 |
| Valine | 388.2 | 356.7 | 368.7 |
| Methionine | 35.4 | 27.3 | 31.0 |
| Total [µmol/g] | 6734.3 | 6158.7 | 6314.5 |

The amino acid analysis of the virgin hair results in the data listed in column. 4a).

Results on the analysis of the hair samples dyed by the non-inventive colorant is given by column 4e). Results on the analysis of the hair samples dyed by a colorant to which the inventive composition was added is given by column 4e).

The result from the hair color test qualitatively confirms the positive result obtained from the bleach trials. The addition of the inventive composition to the hair colorant improves the result of total concentration of amino acids 6158.7 µmol/g for the conventionally dyed hair; dyeing with the inventive composition results in a total concentration of 6314.5 µmol/g. The beneficial analytical results listed in the table were also confirmed by tactile and visual analysis by hair stylists indicating that the inventive treatment was a significant improvement at equal color performance.

### Example 5: Permanent waving

A conventional composition of a permanent wave lotion was used. The composition has the following ingredients:

| | |
|---|---|
| Ammonium Thioglycolate | 9.10% |
| Ammonium Bicarbonate | 2.20% |
| PEG-35 Castor Oil | 0.80% |
| Polyquaternium-6 | 0.40% |
| Ammonia | 0.40% |
| Polyquaternium-16 | 0.20% |
| Propylene Glycol | 0.20% |
| Laureth-9 | 0.09% |
| Caramel | 0.01% |
| Fragrance | 0.30% |
| Water | add up to 100.00% |

A number of 10 well trained hair stylists tested the impact of the inventive composition on 10 model heads. Depending on the length and amount of hair an adequate quantity of the reducing lotion was prepared. The amounts of the inventive composition added to the perm lotion were varied depending on the waving technique and the quality of the hair (thin/thick, hair damage); amounts varied between 5 and 35 %, but was for most between 25% and 35% by weight compared to the total amount of the composition.

The wave lotion comprising the inventive composition produced intensive uniform waves with remarkable bounce. The hair looked very cared and was easy to comb.

For half-head a complarison was made between use of the inventive product in perming applications against the classical application as reference. The tests revealed that the quality of the permed hair got a better overall rating by 9 of 10 hair stylists; for one of 10 models rating was equal to the reference. In detail:

| | |
|---|---|
| Suppleness: | better rating in 8 of 10 cases |
| Bounce: | better rating in 9 of 10 cases |
| Shine: | better rating in 9 of 10 cases, one equal to the reference |
| Combability: | at least equal to reference in 10 of 10 cases |
| Overall curl quality: | natural look combined with excellent bounce |

For a comparative evaluation Olaplex* as market reference for repairing and strengthening hair could not be used together with the permanent wave lotion. Reason is that the active ingredient of Olaplex* is incompatible with keratin reducing agents comprising thiol groups - which was expected based on DE4300320A1.

## Claims

1. A ready-to-use keratin fiber treatment composition, comprising a cosmetic concentrate composition, wherein the keratin fiber treatment composition is a ready-to-use hair color composition or a ready-to-use hair bleach composition wherein the cosmetic concentrate composition concentration is from 2% to 8% by weight of the keratin fiber treatment composition or a ready-to-use permanent hair wave composition wherein the cosmetic concentrate composition concentration is from 5% to 35%, preferably from 25% to 35% by weight of said keratin fiber treatment composition, wherein said concentrate composition comprises at least 2 components, both in an effective amount
- a thiol, which is present at a lower concentration by weight of the concentrate composition from 0.01%, preferably from 0.1%, and at an upper concentration by weight of the concentrate composition of up to 10%, and
- a polymer and/or copolymer comprising at least one cystine unit, which polymer and/or copolymer component is present at a lower concentration by weight of the concentrate composition from 0.1% and at an upper concentration by weight of the concentrate composition up to 3%, and
wherein the polymer and/or copolymer is Cystine Bis-PG-Propyl Silanetriol.

2. A keratin fiber treatment composition according to claim 1 wherein the thiol compound is selected from the group consisting of thioglycolic acid and derivatives thereof, thiolactic acid and derivatives thereof, 3-mercaptopropionic acid and derivatives thereof, cysteamine and derivatives thereof, mono-thioglycerol and derivatives thereof, cysteine and derivatives thereof, and combinations thereof.

3. A keratin fiber treatment composition according to claim 2 wherein a preferred thiol compound is selected from esters of thioglycolic acid preferably glycerol- or glycolmonothioglycolate, esters of thiolactic acid preferably glycerol monothiolactate, esters of 3- mercaptopropionic acid preferably glycerol 3-mercaptopropionate or ethyleneglycol 3-mercaptopropionate, C1.4 acyl derivatives of cysteamine preferably N-acetylcysteamine or N- propionylcysteamine, esters of mono thioglycerol, esters of cysteine preferably N-acetylcysteine ester or N-alkanoylcysteine ester or cysteine alkyl ester, amino acid cystein and its salts, and combinations thereof.

4. A keratin fiber treatment according to any of the preceding claims having a pH in the range from 2 to 12, preferably from 6 to 10, and more preferably from 7.5 to 9.5.

5. A keratin fiber treatment composition according to any of the preceding claims wherein the thiol compound is present at lower concentration by weight of the concentrate composition from 1.0% and at an upper concentration by weight of the concentrate composition of up to 5.0% and preferably up to 4.0%.

6. A keratin fiber treatment composition according to any of the preceding claims wherein said polymer and/or copolymer, comprising at least one cystine unit, is present at a lower concentration by weight of the concentrate composition from 0.5% and at an upper concentration by weight of the concentrate composition up to 1.5%.

7. A keratin fiber treatment composition according to any of the preceding claims wherein said concentrate composition further comprises one or more peptides, preferably selected from an oligopeptide, a polypeptide, a macropeptide, a protein hydrolysate isolated from wheat, oat, soy, silk pr keratin or combinations thereof, from 0.5% and at an upper concentration by weight of the concentrate composition up to 1.5%.

8. A keratin fiber treatment composition according to any of the preceding claims wherein said concentrate composition further comprising compounds useful in cosmetic compositions such as alkali metal sulfites, viscosity regulating agents, pH regulating agents, cationic polymers, preferably Polyquaterniums (INCI name), solvents of the polyol and polyol-ether type, anionic, nonionic and cationic emulsifiers or combinations thereof.

9. A kit for providing a ready-to-use hair color composition according to claim 1 comprising
- a first container containing a cosmetic concentrate composition according to any of the claims 1 to 8
- a second container capable of discharging a second composition by spraying and containing said second composition, said second composition comprising magnesium sulfate in an amount of from 0.1% to about 10% by weight of said second composition, and
- a third container containing a third component comprising a ceramide in a concentration from 0.05% to 0.2% by weight of the ready-to-use hair color composition, a plant extract in a concentration from 0.001% to 0.05% by weight of the ready-to-use hair color composition, a hydrolyzed protein in a concentration from 0.01% to 0.1% by weight of the ready-to-use hair color composition, or a quaternary compound in a concentration from 1% to 10% by weight of the ready-to-use hair color composition and mixtures thereof.

10. Use of a cosmetic concentrate composition in a ready-to-use keratin fiber treatment composition to reduce hair damage during treatment by adding said concentrate composition to the keratin fiber treatment composition at a concentration of 2% to 35% by weight of said keratin fiber treatment composition,
said keratin fiber treatment composition being an alkaline hair dye composition or an alkaline hair bleach composition or a permanent alkaline hair wave composition,
said concentrate composition comprises at least 2 compounds, both in an effective amount to reduce hair damage during treatment,
- a thiol, which is present at a lower concentration by weight of the concentrate composition from 0.01%, preferably from 0.1%, and at an upper concentration by weight of the concentrate composition of up to 10%, and
- a polymer and/or copolymer comprising at least one cystine unit, which polymer and/or copolymer component is present at a lower concentration by weight of the concentrate composition from 0.1% and at an upper concentration by weight of the concentrate composition up to 3%, and
wherein the cystine unit of the polymer and/or copolymer is Cystine Bis-PG-Propyl Silanetriol.

## Patentansprüche

1. Gebrauchsfertige Keratinfaser-Behandlungszusammensetzung, umfassend eine kosmetische Konzentratzusammensetzung, wobei die Keratinfaser-Behandlungszusammensetzung eine gebrauchsfertige Haarfärbezusammensetzung oder eine gebrauchsfertige Haarbleichzusammensetzung ist, wobei die Konzentration der kosmetischen Konzentratzusammensetzung 2 bis 8 Gew.-% der Keratinfaser-Behandlungszusammensetzung beträgt, oder eine gebrauchsfertige Dauerwellenzusammensetzung, wobei die Konzentration der kosmetischen Konzentratzusammensetzung 5 bis 35 Gew.-%, vorzugsweise 25 bis 35 Gew.-% der Keratinfaser-Behandlungszusammensetzung beträgt, und wobei die Konzentratzusammensetzung mindestens 2 Komponenten beide in einer wirksamen Menge, umfasst:
- ein Thiol, das in einer unteren Konzentration, bezogen auf das Gewicht der Konzentratzusammensetzung, von 0,01 %, vorzugsweise von 0,1 %, und in einer oberen Konzentration, bezogen auf das Gewicht der Konzentratzusammensetzung, von bis zu 10 % vorhanden ist, und
- ein Polymer und/oder Copolymer, umfassend mindestens eine Cystineinheit, wobei die Polymer- und/oder Copolymerkomponente in einer niedrigeren Gewichtskonzentration der Konzentratzusammensetzung von 0,1 % und in einer oberen Gewichtskonzentration der Konzentratzusammensetzung von bis zu 3 % vorhanden ist und
wobei die Cystin-Einheit des Polymers und/oder Copolymers Cystin Bis-PG-Propyl Silanetriol ist.

2. Eine Keratinfaser-Behandlungszusammensetzung nach Anspruch 1, wobei die Thiolkomponente ausgewählt ist aus der Gruppe bestehend aus Thioglykolsäure und Derivaten davon, Thiomilchsäure und Derivaten davon, 3-Mercaptopropionsäure und Derivaten davon, Cysteamin und Derivaten davon, Mono-Thioglycerin und Derivaten davon, Cystein und Derivaten davon sowie Kombinationen davon.

3. Eine Keratinfaser-Behandlungszusammensetzung nach Anspruch 2, wobei eine bevorzugte Thiolverbindung ausgewählt ist aus Estern der Thioglykolsäure, vorzugsweise Glycerin- oder Glykolmonothioglykolat, Estern der Thiomilchsäure, vorzugsweise Glycerinmonothiolaktat, Estern der 3-Mercaptopropionsäure, vorzugsweise Glycerin-3-mercaptopropionat oder Ethylenglykol-3-mercaptopropionat, C1. 4-Acylderivate von Cysteamin, vorzugsweise N-Acetylcysteamin oder N-Propionylcysteamin, Ester von Mono-Thioglycerin, Ester von Cystein, vorzugsweise N-Acetylcysteinester oder N-Alkanoylcysteinester oder Cysteinalkylester, Aminosäurecystein und seine Salze sowie Kombinationen davon.

4. Eine Keratinfaserbehandlung nach einem der vorhergehenden Ansprüche mit einem pH-Wert im Bereich von 2 bis 12, vorzugsweise von 6 bis 10 und besonders bevorzugt von 7,5 bis 9,5.

5. Eine Keratinfaser-Behandlungszusammensetzung nach einem der vorhergehenden Ansprüche, wobei die Thiolkomponente in einer unteren Konzentration, bezogen auf das Gewicht der Konzentratzusammensetzung, von 1,0 % und in einer oberen Konzentration, bezogen auf das Gewicht der Konzentratzusammensetzung, von bis zu 5,0 % und vorzugsweise bis zu 4,0 % vorhanden ist.

6. Eine Keratinfaser-Behandlungszusammensetzung nach einem der vorhergehenden Ansprüche, wobei das Polymer und/oder Copolymer, das mindestens eine Cystineinheit umfasst, in einer unteren Gewichtskonzentration der Konzentratzusammensetzung von 0,5 % und in einer oberen Gewichtskonzentration der Konzentratzusammensetzung von bis zu 1,5 % vorhanden ist.

7. Eine Keratinfaser-Behandlungszusammensetzung nach einem der vorhergehenden Ansprüche, wobei die Konzentratzusammensetzung ferner ein oder mehrere Peptide, vorzugsweise ausgewählt aus einem Oligopeptid, einem Polypeptid, einem Makropeptid, einem Proteinhydrolysat, das aus Weizen, Hafer, Soja, Seide, Keratin oder Kombinationen davon isoliert ist, in einer Konzentration von 0,5 Gew.-% und einer oberen Konzentration der Konzentratzusammensetzung bis zu 1,5 Gew.-% enthält.

8. Eine Keratinfaser-Behandlungszusammensetzung nach einem der vorhergehenden Ansprüche, wobei die Konzentratzusammensetzung weiterhin Verbindungen enthält, die in kosmetischen Zusammensetzungen nützlich sind, wie Alkalimetallsulfite, Viskositätsregulierungsmittel, pH-Regulierungsmittel, kationische Polymere, vorzugsweise Polyquaternien (INCI-Name), Lösungsmittel vom Polyol- und Polyol-Ether-Typ, anionische, nichtionische und kationische Emulgatoren oder Kombinationen davon.

9. Kit zur Bereitstellung einer gebrauchsfertigen Haarfärbezusammensetzung nach Anspruch 1, umfassend
- einen ersten Behälter, der eine kosmetische Konzentratzusammensetzung nach einem der Ansprüche 1 bis 8 enthält
- einen zweiten Behälter, der in der Lage ist, eine zweite Zusammensetzung durch Sprühen abzugeben, und der die zweite Zusammensetzung enthält, wobei die zweite Zusammensetzung Magnesiumsulfat in einer Menge von 0,1 bis etwa 10 Gew.-% der zweiten Zusammensetzung umfasst, und
- einen dritten Behälter, der eine dritte Komponente enthält, die ein Ceramid in einer Konzentration von 0,05 Gew.-% bis 0,2 Gew.-% der gebrauchsfertigen Haarfärbezusammensetzung, einen Pflanzenextrakt in einer Konzentration von 0,001 Gew.-% bis 0,05 Gew.-% der gebrauchsfertigen Haarfärbezusammensetzung, ein hydrolysiertes Protein in einer Konzentration von 0,01 Gew.-% bis 0,1 Gew.-% der gebrauchsfertigen Haarfärbezusammensetzung oder eine quaternäre Verbindung in einer Konzentration von 1 Gew.-% bis 10 Gew.-% der gebrauchsfertigen Haarfärbezusammensetzung und Mischungen davon umfasst.

10. Verwendung einer kosmetischen Konzentratzusammensetzung in einer gebrauchsfertigen Keratinfaser-Behandlungszusammensetzung zur Verringerung von Haarschäden während der Behandlung durch Zugabe der Konzentratzusammensetzung zu der Keratinfaser-Behandlungszusammensetzung in einer Konzentration von 2 bis 35 Gew.-% der Keratinfaser-Behandlungszusammensetzung,
wobei die Keratinfaser-Behandlungszusammensetzung eine alkalische Haarfärbezusammensetzung oder eine alkalische Haarbleichzusammensetzung oder eine dauerhafte alkalische Haarwellenzusammensetzung ist,
wobei die Konzentratzusammensetzung mindestens 2 Verbindungen enthält, beide in einer wirksamen Menge, um die Haarschädigung während der Behandlung zu reduzieren,
- ein Thiol, das in einer unteren Konzentration, bezogen auf das Gewicht der Konzentratzusammensetzung, von 0,01 %, vorzugsweise von 0,1 %, und in einer oberen Konzentration, bezogen auf das Gewicht der Konzentratzusammensetzung, von bis zu 10 % vorhanden ist, und
- ein Polymer und/oder Copolymer, umfassend mindestens eine Cystineinheit, wobei die Polymer- und/oder Copolymerkomponente in einer niedrigeren Gewichtskonzentration der Konzentratzusammensetzung von 0,1 % und in einer oberen Gewichtskonzentration der Konzentratzusammensetzung von bis zu 3 % vorhanden ist, und
wobei die Cystin-Einheit des Polymers und/oder Copolymers Cystin Bis-PG-Propyl Silanetriol ist.

## Revendications

1. Composition de traitement de fibres kératiniques prête à l'emploi, comprenant une composition concentrée cosmétique, dans laquelle la composition de traitement de fibres kératiniques est une composition de coloration des cheveux prête à l'emploi ou une composition de décoloration des cheveux prête à l'emploi dans laquelle la concentration de la composition concentrée cosmétique est de 2 % à 8 % en poids de la composition de traitement de fibres kératiniques ou une composition d'ondulation des cheveux permanente prête à l'emploi dans laquelle la concentration de la composition concentrée cosmétique est de 5 % à 35 %, de préférence de 25 % à 35 % en poids de ladite composition de traitement de fibres kératiniques, dans laquelle ladite composition concentrée comprend au moins 2 composants, tous deux en quantité efficace
- un thiol, qui est présent à une concentration inférieure en poids de la composition concentrée de 0,01 %, de préférence de 0,1 % et à une concentration supérieure en poids de la composition concentrée allant jusqu'à 10 %, et
- un polymère et/ou un copolymère comprenant au moins un motif cystine, lequel composant polymère et/ou copolymère est présent à une concentration inférieure en poids de la composition concentrée de 0,1 % et à une concentration supérieure en poids de la composition concentrée allant jusqu'à 3 %, et
dans laquelle le polymère et/ou le copolymère est le cystine bis-PG-propylsilanetriol.

2. Composition de traitement de fibres kératiniques selon la revendication 1 dans laquelle le composé thiol est sélectionné dans le groupe constitué par l'acide thioglycolique et des dérivés de celui-ci, l'acide thiolactique et des dérivés de celui-ci, l'acide 3-mercaptopropionique et des dérivés de celui-ci, la cystéamine et des dérivés de celle-ci, le mono-thioglycérol et des dérivés de celui-ci, la cystéine et des dérivés de celle-ci, et des combinaisons de ceux-ci.

3. Composition de traitement de fibres kératiniques selon la revendication 2 dans laquelle un composé thiol préféré est sélectionné parmi les esters d'acide thioglycolique, de préférence le monothioglycolate de glycérol ou de glycol, les esters d'acide thiolactique, de préférence le monothiolactate de glycérol, les esters de l'acide 3-mercaptopropionique, de préférence le 3-mercaptoproprionate de glycérol ou le 3-mercaptoproprionate d'éthylène glycol, les dérivés acyliques en C1.4 de la cystéamine, de préférence la N-acétyl-cystéamine ou la N-propionylcystéamine, les esters de monothioglycérol, les esters de cystéine, de préférence un ester de N-acétylcystéine ou un ester de N-alcanoylcystéine ou un ester alkylique de cystéine, l'acide aminé cystéine et ses sels, et des combinaisons de ceux-ci.

4. Traitement de fibres kératiniques selon l'une quelconque des revendications précédentes ayant un pH compris dans la plage allant de 2 à 12, de préférence de 6 à 10, et plus préférablement de 7,5 à 9,5.

5. Composition de traitement de fibres kératiniques selon l'une quelconque des revendications précédentes dans laquelle le composé thiol est présent à une concentration inférieure en poids de la composition concentrée de 1,0 % et à une concentration supérieure en poids de la composition concentrée allant jusqu'à 5,0 % et de préférence allant jusqu'à 4,0 %.

6. Composition de traitement de fibres kératiniques selon l'une quelconque des revendications précédentes dans laquelle ledit polymère et/ou copolymère, comprenant au moins un motif cystine, est présent à une concentration inférieure en poids de la composition concentrée de 0,5 % et à une concentration supérieure en poids de la composition concentrée allant jusqu'à 1,5 %.

7. Composition de traitement de fibres kératiniques selon l'une quelconque des revendications précédentes dans laquelle ladite composition concentrée comprend en outre un ou plusieurs peptides, de préférence sélectionnés parmi un oligopeptide, un polypeptide, un macropeptide, un hydrolysat de protéine isolé à partir de blé, d'avoine, de soja pr kératine ou une combinaison de ceux-ci. de 0,5 % et à une concentration supérieure en poids de la composition concentrée allant jusqu'à 1,5 %.

8. Composition de traitement de fibres kératiniques selon l'une quelconque des revendications précédentes dans laquelle ladite composition concentrée comprend en outre des composés utiles dans les compositions cosmétiques tels que des sulfites de métal alcalin, des agents de régulation de la viscosité, des agents de régulation du pH, des polymères cationiques, de préférence des polyquaterniums (nom INCI), des solvants de type polyol et éther de polyol, des émulsifiants anioniques, non ioniques et cationiques ou des combinaisons de ceux-ci.

9. Kit permettant de fournir une composition de coloration des cheveux prête à l'emploi selon la revendication 1 comprenant
- un premier récipient contenant une composition concentrée cosmétique selon l'une quelconque des revendications 1 à 8
- un deuxième récipient capable de faire sortir une deuxième composition par pulvérisation et contenant ladite deuxième composition, ladite deuxième composition comprenant du sulfate de magnésium en une quantité de 0,1 % à environ 10 % en poids de ladite deuxième composition, et
- un troisième récipient contenant un troisième composant comprenant un céramide en une concentration de 0,05 % à 0,2 % en poids de la composition de coloration des cheveux prête à l'emploi, un extrait végétal en une concentration de 0,001 % à 0,05 % en poids de la composition de coloration des cheveux prête à l'emploi, une protéine hydrolysée en une concentration de 0,01 % à 0,1 % en poids de la composition de coloration des cheveux prête à l'emploi, ou un composé quaternaire en une concentration de 1 % à 10 % en poids de la composition de coloration des cheveux prête à l'emploi et des mélanges de ceux-ci.

10. Utilisation d'une composition concentrée cosmétique dans une composition de traitement de fibres kératiniques prête à l'emploi pour réduire l'endommagement des cheveux pendant le traitement par ajout de ladite composition concentrée à la composition de traitement de fibres kératiniques à une concentration de 2 % à 35 % en poids de ladite composition de traitement de fibres kératiniques,
ladite composition de traitement de fibres kératiniques étant une composition de coloration des cheveux alcaline ou une composition de décoloration des cheveux alcaline ou une composition d'ondulation des cheveux alcaline permanente, ladite composition concentrée comprend au moins 2 composés, tous deux en quantité efficace pour réduire l'endommagement des cheveux pendant le traitement,
- un thiol, qui est présent à une concentration inférieure en poids de la composition concentrée de 0,01 %, de préférence de 0,1 % et à une concentration supérieure en poids de la composition concentrée allant jusqu'à 10 %, et
- un polymère et/ou un copolymère comprenant au moins un motif cystine, lequel composant polymère et/ou copolymère est présent à une concentration inférieure en poids de la composition concentrée de 0,1 % et à une concentration supérieure en poids de la composition concentrée allant jusqu'à 3 %, et
dans laquelle le motif cystine du polymère et/ou copolymère est le cystine bis-PG-propylsilanetriol.
